# EUROPÄISCHE PATENTANMELDUNG

(11) **EP 1 449 526 A1**
(43) Veröffentlichungstag der Anmeldung: **25.08.2004**
(21) Anmeldenummer: 03003888.9
(22) Anmeldetag: 21.02.2003
(51) Int. Cl.: A61K 9/70

(54) **UV-stabiles transdermales Pflaster**

(71) Anmelder: Schering AG, 13342 Berlin (DE)
(72) Erfinder: Langguth, Thomas, 07747 Jens (DE); Schumacher, Jochen, 07751 Bucha (DE); Süsse, Manfred, 07381 Langenorla (DE); Dittgen, Michael, 99510 Apolda (DE); Mletzko, Stephan, 14053 Berlin (DE); Ingwersen, Jan-Peter, 13465 Berlin (DE); Schenk, Dirk, 83623 Dietramszell (DE); Kaffl, Hubert, 83730 Fischbachau (DE)
(74) Vertreter: Cramer, Eva-Maria

(57) **Zusammenfassung**

Die Erfindung betrifft ein neues UV-stabiles transdermales therapeutisches System (TTS), welches aus einer Rückschicht, mindestens einer wirkstoffhaltigen Matrix und wahlweise einer Abziehfolie besteht sowie einen UV-Absorber enthält, wobei zwischen der Rückschicht und der der Hautoberfläche am weitesten abgewendeten wirkstoffhaltigen Matrix mindestens eine UV-Absorber enthaltende Klebschicht vorgesehen ist und zwischen der den UV-Absorber enthaltenden Klebschicht und der der Hautoberfläche am weitesten abgewendeten wirkstoffhaltigen Matrix mindestens eine Trennschicht enthalten ist, die wirkstoffundurchlässig und für den UV-Absorber undurchlässig ist.

Das erfindungsgemäße transdermale therapeutische System realisiert eine hohe Stabilität ohne die Nachteile herkömmlicher lichtempfindliche Wirkstoffe enthaltende TTS.

## Beschreibung

Die Erfindung betrifft ein neues UV-stabiles transdermales therapeutisches System (TTS), welches aus einer Rückschicht, mindestens einer wirkstoffhaltigen Matrix und wahlweise einer Abziehfolie besteht sowie einen UV-Absorber enthält, wobei zwischen der Rückschicht und der der Hautoberfläche am weitesten abgewendeten wirkstoffhaltigen Matrix mindestens eine UV-Absorber enthaltende Klebschicht vorgesehen ist und zwischen der den UV-Absorber enthaltenden Klebschicht und der der Hautoberfläche am weitesten abgewendeten wirkstoffhaltigen Matrix mindestens eine Trennschicht enthalten ist, die wirkstoffundurchlässig und für den UV-Absorber undurchlässig ist.

Es sind Versuche bekannt, lichtempfindliche Wirkstoffe, welche UV-Aund UV-B-Strahlen absorbieren und üblicherweise in Sonnencremes einzusetzen, wie von Briscart & Plaizier-Vercammen (Proc. 2^{nd} World Meeting on Pharmaceutics, Biopharmaceutics and Pharmaceutical Technology, APGI/ APV, 1998, 1231-1232) beschrieben.
Ferner ist bekannt, mit lichtempfindlichen Wirkstoffen versehene transdermale therapeutische Systeme mittels optisch auffallenden aluminisierten oder lackierten Abdeckfolien als Rückschicht des TTS zu schützen. Die DE-A1-19912623 beschreibt ein Verfahren zum Schutz therapeutischer Zubereitungen, Systeme oder deren Bestandteile, wobei ein jeweils spezifischer Schutz gegen Abbau durch schädliche Faktoren, wie Luftsauerstoff, Wasser und/oder Licht realisiert werden soll. Es werden elektromagnetische Wellen absorbierende bzw. reflektierende Lichtschutzsubstanzen verwendet, wobei Absorptions- bzw. Reflexionsmittel eingesetzt werden, deren Absorptions- bzw. Reflekionsspektrum denjenigen Wellenlängenbereich umfasst, der für die Instabilität des lichtempfindlichen Stoffes bzw. seiner Bestandteile verantwortlich ist. Als Abdeckfolie werden hier u.a. eingefärbte Kunststofffolien verwendet, am Beispiel vom 1,4-Dihydopyridinderivat Lacidipin aufgezeigt.
Die Einfärbung von hochflexiblen Kunststofffolien erweist sich als schwierig und bietet durch häufig auftretende Risse in der Farbschicht der Kunststofffolie keinen zuverlässigen Lichtschutz.

Ferner sind aus DE-C1-10053375 transdermale therapeutische Systeme (TTS) bekannt, die aus einer wirkstoffhaltigen Polymermatrix und einer Rückschicht bestehen, wobei Polymermatrix und Rückschicht fest verbunden sind bzw. ein Laminat bilden und sowohl die Polymermatrix als auch die Rückschicht eine im UV-Bereich absorbierende, farblose Substanz enthalten, die keine eigene pharmakologische Wirkung aufweist. Nachteilig an dieser Lösung ist,
- dass in der Polymermatrix gegebenenfalls durch Wechselwirkung der im UV-Bereich absorbierenden farblosen Substanz mit dem Wirkstoff eine nicht erwünschte Beeinflussung der Stabilität des TTS zu verzeichnen ist,
- dass durch die feste Verbindung zwischen Polymermatrix und wirkstoffdurchlässiger Rückschicht eine hohe unzulässige Diffusion von Wirkstoff aus der Polymermatrix in die Rückschicht erfolgen kann, hauptsächlichst bei Rückschichten aus Polypropylen, Polyethylen oder Polyurethan und schliesslich auf der Oberseite der Rückschicht/Abdeckfolie austreten oder auskristallisieren kann,
- dass durch direkten Hautkontakt mit den im UV-Bereich absorbierenden Substanzen, welche sich in der Rückschicht/Abdeckfolie befinden, Irritationen der Haut hervorgerufen werden können.

Aufgabe der Erfindung ist deshalb, eine mit einem lichtempfindlichen Wirkstoff versehene transdermal zu applizierende Arzneistoffzubereitung bereitzustellen, welche eine hohe Stabilität ohne die vorher genannten Nachteile realisiert.

Erfindungsgemäß wird die Aufgabe durch ein transdermales therapeutisches System (TTS), bestehend aus einer Rückschicht, mindestens einer wirkstoffhaltigen Matrix und wahlweise einer Abziehfolie sowie enthaltend einen UV-Absorber gelöst, wobei zwischen der Rückschicht und der der Hautoberfläche am weitesten abgewendeten wirkstoffhaltigen Matrix mindestens eine UV-Absorber enthaltende Klebschicht vorgesehen ist und zwischen der den UV-Absorber enthaltenden Klebschicht und der der Hautoberfläche am weitesten abgewendeten wirkstoffhaltigen Matrix mindestens eine Trennschicht enthalten ist, die wirkstoffundurchlässig und für den UV-Absorber undurchlässig ist.

Erfindungsgemäß kann das transdermale therapeutische System von der hautabgewandten Seite her die Schichtenabfolge Rückschicht, UV-absorberhaltige Klebschicht, Trennschicht und schließlich eine ein- oder zweischichtige wirkstoffhaltige Matrix aufweisen, deren haftklebende Oberfläche mit einer abziehbaren Schutzfolie abgedeckt ist.
Ferner kann erfindungsgemäß bei dem transdermalen therapeutischen System die Trennschicht eine Schichtdicke von 4 bis 23 µm, vorzugsweise von 4 bis 10 µm, aufweisen und aus einem Barrierepolymer bestehen. Als Barrierepolymere eignen sich Polyethylenterephthalat, Polyacrylnitril, Polyvinylchlorid, Polyvinylidenchlorid oder dessen Copolymere oder Kolaminate.Bei dem erfindungsgemäßen transdermalen therapeutischen System kann die Matrix selbstklebend ausgeführt sein und keine die Wirkstofffreisetzung steuernde Membran aufweisen, sowie im wesentlichen aus Polymeren bestehen, die aus der Gruppe Polyisobutylen, Polybuten, Polyacrylat, Polydimethylsiloxan, Styrol-Isopren-Blockpolymerisat oder Polyisopren ausgewählt sind. Das Flächengewicht der Matrix kann erfindungsgemäß 30 bis 150 g/m², vorzugsweise 50 bis 120 g/m² , besonders bevorzugt etwa 100 g/m² betragen.

Bei dem erfindungsgemäßen transdermalen therapeutischen System kann die Rückschicht eine transparente Folie aus der Gruppe Polypropylen, Polyethylen, Polyurethan, Polyester, Ethylen-Vinylacetat-Copolymer oder Polyethylenterephthalat oder deren Mischungen sein und kann wirkstoffdurchlässig sein.

Ferner kann bei dem erfindungsgemäßen transdermalen therapeutischen System in der Klebschicht der UV-Absorber in einer Konzentration von 0,5 bis 10 % (m/m), vorzugsweise 1,0 bis 5,0 % (m/m), besonders bevorzugt 2,0 bis 4,0 % (m/m) in gelöster Form vorliegen, die Klebschicht selbstklebend ausgeführt sein und im wesentlichen aus Polymeren bestehen, die aus der Gruppe Polyisobutylen, Polybuten, Polyacrylat, Polydimethylsiloxan, Styrol-Isopren-Blockpolymerisat oder Polyisopren ausgewählt sind. Ferner kann die Klebschicht ein Flächengewicht von 15 bis 30 g/m², vorzugsweise 20 bis 25 g/m² besitzen.

Auch kann bei dem erfindungsgemäßen transdermalen therapeutischen System die Klebschicht ausschließlich den/die UV-Absorber aufweisen, wobei diese farblos oder gelblich sein können.

Weiterhin kann bei dem erfindungsgemäßen transdermalen therapeutischen System die Klebschicht einen UV-Absorbergehalt aus einem Gemisch von zwei oder mehreren im UV-Bereich absorbierenden Substanzen aufweisen, wobei der/die UV-Absorber aus der Gruppe p-Aminobenzoesäure, Aminobenzoesäurederivat, vorzugsweise 4-Dimethylaminobenzoesäure-2-ethyl-hexylester und/oder 4-Bis-(polyethoxyl)aminobenzoesäure-polyethoxyethylester, Zimtsäure,Zimtsäure-Derivate, vorzugsweise 4-Methoxylzimt-säureisoamylester und/oder 4-Methoxyzimtsäure-2-ethylhexyl-ester, 3-Benzylidenbornan-2-on, Benzylidenbornan-2-on-Derivate, vorzugsweise 3-(4')-Methylbenzylinden-bornan-2-on, 3-(4-Sulfon)benzylidenbornan-2-on und/oder 3-(4'-Trimethyl-ammonium)benzylidenbornan-2-onmethylsulfat, Salicylsäurederivat, vorzugsweise 4-Isopropylbenzylsalicylat, Salicysäure-2-ethylhexylester, und/oder 3, 3, 5-Trimethyl-cyclohexylsalicylat, Benzotriazole, vorzugsweise 2-(5-chloro-2H-benzotriazole-2-yl)-6-(1,1-dimethylethyl)-4-methyl-phenol, 2, 4 ,6'-Trianilin-p-(carbo-2'-ethylhexyl-1'-oxy)-1, 3, 5-triazin, 3-Imidazol-4-yl-acrylsäure, 3-Imidazol-4-yl-.3-Imidazol-4-yl-acrylsäure-Ester, 2-Phenylenbenzimidazol-5-sulfonsäure und/oder ihre K-, Na- und Triethanolamin (=TEA)-Salze, 2-Cyan-3,3-diphenylacrylsäure, Terephthaloyliden-di-campher-sulfonsäure, Butylmethoxy-dibenzoyl-methan; Benzophenon und/oder Benzophenon-Derivate, vorzugsweise Benzophenon-3 und/oder Benzophenon-4 ausgewählt sein kann/können. Ferner kann/können der/die UV-Absorber farblos oder gelblich sein.
Weiterhin kann das erfindungsgemäße transdermalen therapeutischen System transparent oder schwach opak sein.

Bei dem erfindungsgemäßen transdermalen therapeutischen System kann als pharmazeutischer Wirkstoff mindestens ein Hormon wirksam werden und Gestagen/e, vorzugsweise Gestoden oder Levonorgestrel, sein.

Das erfindungsgemäße transdermale therapeutische System besitzt gegegenüber herkömmlichen Systemen mit lichtempfindlichen Wirkstoffgehalt folgende Vorteile:
- Indem man die Dicke der den UV-Absorber enthaltene Schicht oder die Konzentration des UV-Absorbers in derselben variiert, kann der gewünschte UV-Schutz genau eingestellt werden. Dies ist ein wesentlicher Vorteil gegenüber der Verwendung herkömmlicher TTS mit eingearbeitetem UV-Schutz.
- Ein Kontakt zwischen dem UV-Absorber mit dem Wirkstoff bzw. den Wirkstoffen in der wirkstoffhaltigen Matrix wird ausgeschlossen, so dass weder der UV-Absorber noch dessen Abbauprodukte, die unter Lichteinfluss entstehen können, mit dem bzw. den Wirkstoffen reagieren können.
- Wird eine Abdeckfolie verwendet, die wirkstoffdurchlässig ist, so kann die Diffusion von Wirkstoff bei der Lagerung des Systems unzulässig hohe Werte erreichen, sodass Wirkstoff auf der Oberseite der Abdeckfolie austreten oder auskristallisieren kann. Diesen Effekt kann man beispielsweise bei Abdeckfolien aus Polypropylen, Polyethylen oder Polyurethan beobachten. Die erfindungsgemäß vorgesehene Trennschicht zwischen der Schicht mit UV-Absorber und der wirkstoffhaltigen Schicht stellt nun eine Barriere gegen einen Wirkstoffverlust durch Diffusion durch die Abdeckfolie dar.

Außerdem kann ein Hautkontakt mit UV-Absorbern und damit eine mögliche Hautirritation vermieden werden.

Die Erfindung und ihre vorteilhaften Eigenschaften werden durch nachfolgende Beispiele näher erläutert.

### Beispiel 1

Es wurden zwei Formulierungen eines lichtempfindlichen Wirkstoffes aus der Gruppe der Gestagene hergestellt. Formulierung l enthält eine Klebeschicht und eine Trennschicht, wobei die Klebeschicht 3Gew.% einer UV-absorbierende Substanz enthält.
Formulierung II enthält keine Klebeschicht und Trennschicht und dient als Vergleichsformulierung. Beide Formulierungen enthalten eine wirkstoffhaltige Matrix mit einem lichtempfindlichem Gestagen und wurden mit einer Rückschicht aus Polyethylen ausgestattet, wodurch jeweils ein TTS erhalten wird.
Die Formulierung l hat folgende Zusammensetzung:
1. wirkstoffhaltige Matrix:
   - 1,9 % Gestagen
   - 98,1 % Polyisobutylen basierendes Klebemittel
2. Klebeschicht:
   - 3 % Tinuvin® 326
   - 97 % Polyisobutylen basierendes Klebemittel
Tinuvin®326 (Fa. CIBA, Lampertheim) ist ein UV-Absorber der Hydroxyphenylbenzotriazol-Klasse.
Zur Untersuchung der Lichtschutzwirkung wurden beide Formulierungen mit Licht mit einem UV-Spektrum von 300 - 800 nm über eine Dauer von bis zu 14h bestrahlt. Als Strahlungsquelle wurde eine Xenon-Lampe verwendet. Zwischen der Strahlungsquelle und den zu bestrahlenden Proben wurde eine Filtersystem (Typ: Suprax®-Filter) gesetzt, um die Bestrahlung unter realistischen Anwendungsbedingungen der TTS nachzuahmen. Anschließend wurde der Wirkstoffgehalt in den TTS bestimmt. Es zeigte sich, dass die TTS der Formulierung A, die eine Klebeschicht mit UV-absorbierender Substanz und eine Trennschicht enthielt, nach Bestrahlung über 14 h noch ca. 99% der ursprünglich eingesetzten Menge des lichtempfindlichen Wirkstoffes enthielten, wogegen die TTS der Formulierung B bereits nach der Bestrahlung von 7h nur noch ca. 24 % der ursprünglich eingesetzten Menge des lichtempfindlichen Wirkstoffs enthielten (Abb.1). Dies zeigt, dass das erfindungsgemäße System unter realistischen Anwendungsbedingungen einen verbesserten Sonnenschutz aufweist, da die UV-Schutzwirkung des erfindungsgemäßen Systems (Formulierung A) wesentlich höher als die des Vergleichssystems (Formulierung B) war.

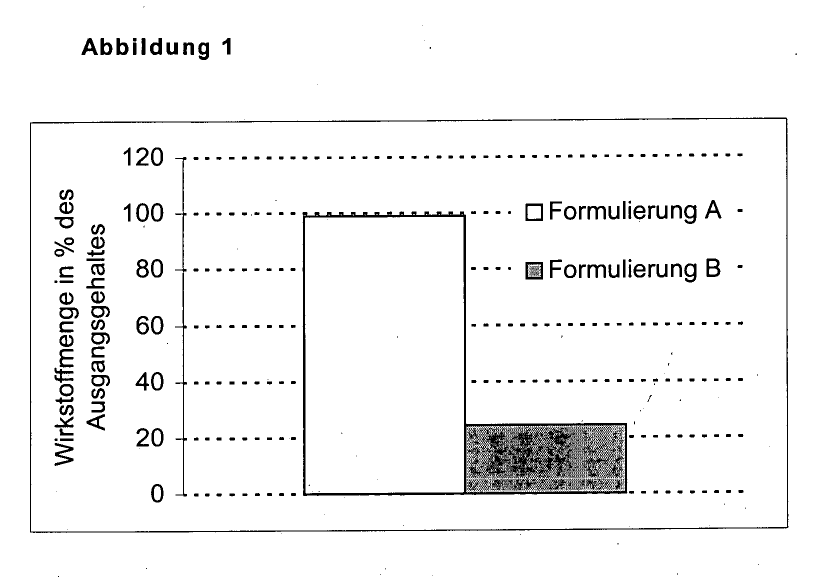

### Beispiel 2

Formulierung mit einem lichtempfindlichen Wirkstoffes aus der Gruppe der Gestagene mit jeweils einer Klebschicht und Trennschicht, bei dem die Trennschichte aus Polyethylenterephthalat (Hostaphan®, Fa. Mitsubishi Polyester, Wiesbaden) besteht.
Die Formulierung hat folgende Zusammensetzung:
1. wirkstoffhaltige Matrix:
   - 1,9 % Gestagen
   - 98,1 % Polyisobutylen basierendes Klebemittel
2. Klebeschicht 1 und 2:
   - 3 % Uvinul®MC80
   - 97 % Polyacrylat basierendes Klebemittel Uvinul®MC 80 (Fa. BASF, Ludwigshafen) ist ein Methoxyzimtsäure-Derivat.

### Beispiel 3

Formulierung mit einem lichtempfindlichen Wirkstoffes aus der Gruppe der Gestagene mit jeweils zwei Klebschichten und Trennschichten, bei dem die Trennschichten aus Polyethylenterephthalat (Hostaphan®, Fa. Mitsubishi Polyester, Wiesbaden) bestehen.
Die Formulierung l hat folgende Zusammensetzung:
3. wirkstoffhaltige Matrix:
   - 1,9 % Gestagen
   - 98,1 % Polyisobutylen basierendes Klebemittel
4. Klebeschicht 1 und 2:
   - 3 % Uvinul®M40
   - 97 % Polyacrylat basierendes Klebemittel
Uvinul®M40 (Fa. BASF, Ludwigshafen) ist ein Benzophenon-Derivat.

## Patentansprüche

1. Transdermales therapeutisches System (TTS),
bestehend aus einer Rückschicht , mindestens einer wirkstoffhaltigen Matrix und wahlweise einer Abziehfolie sowie enthaltend einen UV-Absorber,
**dadurch gekennzeichnet, dass**
- zwischen der Rückschicht und der der Hautoberfläche am weitesten abgewendeten wirkstoffhaltigen Matrix mindestens eine UV-Absorber enthaltene Klebschicht vorgesehen ist,
- zwischen der den UV-Absorber enthaltenden Klebschicht und der der Hautoberfläche am weitesten abgewendeten wirkstoffhaltigen Matrix mindestens eine Trennschicht enthalten ist, die wirkstoffundurchlässig und für den UV-Absorber undurchlässig ist.

2. Transdermales therapeutisches System nach Anspruch 1, **dadurch gekennzeichnet,**
**dass** das System von der hautabgewandten Seite her die Schichtenabfolge Rückschicht, UV-absorberhaltige Klebschicht, Trennschicht und schließlich eine ein- oder zweischichtige wirkstoffhaltige Matrix aufweist, deren haftklebende Oberfläche mit einer abziehbaren Schutzfolie abgedeckt ist.

3. Transdermales therapeutisches System nach mindestens einem der vorhergehenden Ansprüche, **dadurch gekennzeichnet,**
**dass** das Flächengewicht der Klebschicht 15 bis 30 g/m², vorzugsweise 20 bis 25 g/m² beträgt.

4. Transdermales therapeutisches System nach einem oder mehreren der vorangehenden Ansprüche, **dadurch gekennzeichnet,**
**dass** die Trennschicht eine Schichtdicke von 4 bis 23 µm, vorzugsweise von 4 bis 10 µm, aufweist.

5. Transdermales therapeutisches nach mindestens einem der vorhergehenden Ansprüche, **dadurch gekennzeichnet,**
**dass** die Trennschicht aus einem Barrierepolymer besteht, vorzugsweise aus Polyethylenterephthalat, Polyacrylnitril, Polyvinylchlorid, Polyvinylidenchlorid oder dessen Copolymeren oder Kolaminaten.

6. Transdermales therapeutisches nach mindestens einem der vorhergehenden Ansprüche, **dadurch gekennzeichnet,**
**dass** die Matrix und/ oder die Klebschicht selbstklebend ausgeführt ist/ sind und im wesentlichen aus Polymeren besteht/ bestehen, die aus den Gruppen Polyisobutylen, Polybuten, Polyacrylat, Polydimethylsiloxan, Styrol-Isopren-Blockpolymerisat oder Polyisopren ausgewählt ist/sind.

7. Transdermales therapeutisches System nach mindestens einem der vorhergehenden Ansprüche, **dadurch gekennzeichnet,**
**dass** das Flächengewicht der Matrix 30 bis 150 g/m², vorzugsweise 50 bis 120 g/m² beträgt.

8. Transdermales therapeutisches System nach mindestens einem der vorhergehenden Ansprüche, **dadurch gekennzeichnet,**
**dass** die Rückschicht wirkstoffdurchlässig ist und vorzugsweise aus Polypropylen, Polyethylen, Polyurethan, Ethylen-Vinylacetat-Copolymer oder einem Mehrschichtverbund aus diesen Materialien untereinander oder mit anderen Materialien besteht.

9. Transdermales therapeutisches System nach mindestens einem der vorhergehenden Ansprüche, **dadurch gekennzeichnet,**
**dass** in der Klebschicht der UV-Absorber in einer Konzentration von 0,5 bis 10 % (m/m), vorzugsweise 1,0 bis 5,0 % (m/m) in gelöster Form vorliegt .

10. Transdermales therapeutisches System nach mindestens einem der vorhergehenden Ansprüche, **dadurch gekennzeichnet,**
**dass** ausschließlich die Klebschicht den/die UV-Absorber aufweist/aufweisen.

11. Transdermales therapeutisches System nach mindestens einem der vorhergehenden Ansprüche, **dadurch gekennzeichnet,**
**dass** es sich bei dem UV-Absorber um eine Substanz oder eine Mischung von Substanzen aus der Gruppe
p-Aminobenzoesäure, Aminobenzoesäurederivat, vorzugsweise 4-Dimethylaminobenzoesäure-2-ethyl-hexylester und/oder 4-Bis-(polyethoxyl)-aminobenzoesäure-polyethoxyethylester,
Zimtsäure,Zimtsäure-Derivate, vorzugsweise 4-Methoxylzimt-säureisoamylester und/oder 4-Methoxyzimtsäure-2-ethylhexylester,
3-Benzylidenbornan-2-on, Benzylenbornan-2-on-Derivate, vorzugsweise 3-(4')-Methylbenzylinden-bornan-2-on, 3-(4-Sulfon)benzylidenbornan-2-on und/oder 3-(4'-Trimethylammonium)benzylidenbornan-2-on-methylsulfat, Salicylsäurederivat, vorzugsweise 4-Isopropylbenzylsalicylat, Salicysäure-2-ethylhexylester, und/oder 3, 3, 5-Trimethyl-cyclohexylsalicylat,
Benzotriazole, vorzugsweise 2-(5-chloro-2H-benzotriazole-2-yl)-6-(1,1-dimethylethyl)-4-methyl-phenol, 2, 4 ,6'-Trianilin-p-(carbo-2'-ethylhexyl-1'-oxy)-1, 3, 5-triazin, 3-Imidazol-4-ylacrylsäure, 3-Imidazol-4-yl-.3-Imidazol-4-yl-acrylsäure-Ester, 2-Phenylenbenzimidazol-5-sulfonsäure und/oder ihre K-, Na- und Triethanolamin (=TEA)-Salze, 2-Cyan-3,3-diphenylacrylsäure,
Terephthaloyliden-di-campher-sulfonsäure, Butylmethoxy-dibenzoyl-methan,
Benzophenon und/oder Benzophenon-Derivate, vorzugsweise Benzophenon-3 und/oder Benzophenon-4 ausgewählt ist/sind.

12. Transdermales therapeutisches System nach mindestens einem der vorhergehenden Ansprüche, **dadurch gekennzeichnet, dass** der/die UV-Absorber farblos oder gelblich ist/sind.

13. Transdermales therapeutisches System nach mindestens einem der vorhergehenden Ansprüche, **dadurch gekennzeichnet,**
**dass** System transparent oder schwach opak ist.

14. Transdermales therapeutisches System nach mindestens einem der vorhergehenden Ansprüche, **dadurch gekennzeichnet,**
**dass** als Wirkstoff mindestens ein Hormon wirksam wird.

15. Transdermales therapeutisches System nach mindestens einem der vorhergehenden Ansprüche, **dadurch gekennzeichnet,**
**dass** der/die pharmazeutische/en Wirkstoff/e Gestagen/e, vorzugsweise Gestoden oder Levonorgestrel, ist/sind.

16. Transdermales therapeutisches System nach mindestens einem der vorhergehenden Ansprüche, **dadurch gekennzeichnet,**
**dass**, das transdermale therapeutische System keine die Wirkstofffreisetzung steuernde Membran aufweist.
